# EUROPEAN PATENT APPLICATION

(11) **EP 4 502 129 A1**
(43) Date of publication of application: **05.02.2025**
(21) Application number: 23780342.4
(22) Date of filing: 27.03.2023
(51) Int. Cl.: C12C 7/04, A23L 33/00, A23L 33/125, C12N 1/20, C12P 1/06

(54) **FERMENTED FOOD AND PRODUCTION METHOD THEREOF**

(30) Priority: 29.03.2022 JP 2022053741
(71) Applicant: Kabushiki Kaisha Yakult Honsha, Tokyo, 105-8660 (JP)
(72) Inventor: NAITO, Eiichiro, Tokyo 105-8660 (JP); SUZUKI, Chiaki, Tokyo 105-8660 (JP); KONDO, Yu, Tokyo 105-8660 (JP); YOSHIDA, Yasuto, Tokyo 105-8660 (JP)
(74) Representative: Wächtershäuser & Hartz Patentanwaltspartnerschaft mbB
(86) International application number: PCT/JP2023/012184
(87) International publication number: WO 2023/190332

(57) **Abstract**

Using a fermented food containing a lactic acid bacterium, the fermented food being substantially free from glucose, the fermented food containing sugars which can be assimilated by the lactic acid bacterium other than glucose, the sugars which can be assimilated by the lactic acid bacterium being derived from a vegetable material containing starch, the fermented food having a pH of 4.5 or lower and using a method for producing a fermented food, the method including the following steps (a) and (b), a step (a) of culturing a lactic acid bacterium in a medium containing glucose and sugars which can be assimilated by the lactic acid bacterium other than glucose until the medium becomes substantially free from glucose and a step (b) of adjusting the pH to 4.5 or lower when the pH after the termination of the culture is higher than 4.5, the acid production by a lactic acid bacterium is inhibited in a fermented food containing the lactic acid bacterium and sugars, and the flavor and the viability of the lactic acid bacterium are improved.

## Description

### Technical Field

The present invention relates to a fermented food containing a lactic acid bacterium and specific sugars and having a pH of 4.5 or lower and a production method thereof.

### Background Art

With the recent increasing trend toward health of consumers, many fermented drinks, and fermented foods such as yogurt which enable easy intake of effective microorganisms such as lactic acid bacteria are sold.

With negative impression about animal materials and sugars, restrictions on intake of and increased taxation on free sugars (monosaccharides/disaccharides) for the purpose of disease prophylaxis, and the like as backgrounds, there is an increasing need for plant-based drinks, and fermented foods obtained from a liquefied or saccharified vegetable material containing starch such as rice as a fermentation substrate have been developed.

For example, rice contains a large amount of starch and can be processed into a fermentation substrate containing glucose (Glc) and maltose (Mal) through liquefaction or saccharification using *koji,* malt or an enzyme, and these sugars can be used as growth substrates for lactic acid bacteria or sweeteners of drinks. A technique regarding a method for producing a starch-derived yogurt drink obtained from rice flour or the like as a raw material has been actually reported (PTL 1) .

In this technique, however, a liquefied or saccharified fermentation substrate is merely fermented. Thus, due to excessive fermentation depending on the culture conditions of the lactic acid bacterium or the degree of liquefaction or saccharification of the fermentation substrate, the drink has a too strong acid taste, requires addition of a sweetener for adjusting the flavor, or has a significantly decreased count of the lactic acid bacterium due to excessive acid production.

### Citation List

### Patent Literature

PTL 1: JP2016-93151A

### Summary of Invention

### Technical Problem

Accordingly, a problem of the invention is to provide a technique for inhibiting acid production by a lactic acid bacterium and improving the flavor and the viability of the lactic acid bacterium in a fermented food containing the lactic acid bacterium and sugars.

### Solution to Problem

As a result of extensive studies to solve the problem, the present inventors have found that the problem can be solved by culturing a lactic acid bacterium in a medium containing glucose and sugars which can be assimilated by the lactic acid bacterium other than glucose until the medium becomes substantially free from glucose and by adjusting the pH to 4.5 or lower, and the inventors have thus completed the invention.

That is, the invention is a fermented food containing a lactic acid bacterium, the fermented food is substantially free from glucose, the fermented food contains sugars which can be assimilated by the lactic acid bacterium other than glucose, the sugars which can be assimilated by the lactic acid bacterium is derived from a vegetable material containing starch, and the fermented food has a pH of 4.5 or lower.

Moreover, the invention is a method for producing a fermented food, the method including the following steps (a) and (b):
a step (a) of culturing a lactic acid bacterium in a medium containing glucose and sugars which can be assimilated by the lactic acid bacterium other than glucose until the medium becomes substantially free from glucose; and
a step (b) of adjusting the pH to 4.5 or lower when the pH after the termination of the culture is higher than 4.5.

Furthermore, the invention is a method for inhibiting assimilation of sugars which can be assimilated by a lactic acid bacterium other than glucose during storage of a fermented food containing a lactic acid bacterium which is characterized in that
the fermented food is substantially free from glucose,
contains the sugars which can be assimilated by the lactic acid bacterium other than glucose, and
has a pH of 4.5 or lower.

Furthermore, the invention is a method for inhibiting a decrease in the pH during storage of a fermented food containing a lactic acid bacterium which is characterized in that
the fermented food is substantially free from glucose,
contains sugars which can be assimilated by the lactic acid bacterium other than glucose, and
has a pH of 4.5 or lower.

### Advantageous Effects of Invention

The fermented food of the invention has high storage stability because the acid production by a lactic acid bacterium can be inhibited and the flavor and the viability of the lactic acid bacterium can be improved.

Even though the fermented food of the invention has been fermented with a lactic acid bacterium until the fermented food is substantially free from glucose, because the fermented food contains sugars derived from a vegetable material which can be assimilated by the lactic acid bacterium other than glucose, a sweet taste can be felt even without the addition of a sweetener.

Furthermore, the method for producing a fermented food of the invention is a simple production method because it is basically sufficient to control only the glucose amount and the pH of the medium for producing the excellent fermented food with a lactic acid bacterium.

### Brief Description of Drawings

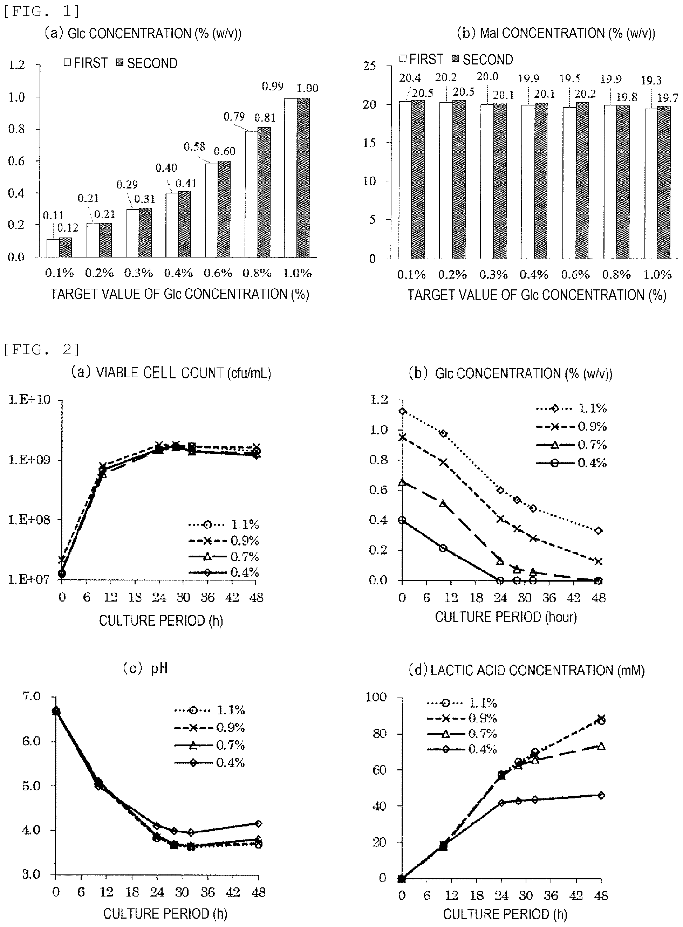

[Fig. 1] Fig. 1 shows the target values and the measured values of the Glc concentrations and the Mal concentrations of the saccharified materials regarding saccharified unpolished rice materials having Glc concentrations of 0.1 to 1.0% (w/v) which were prepared by mixing two kinds of saccharified unpolished rice materials having different Glc concentrations at various ratios in Reference Example 1.
[Fig. 2] Fig. 2 shows the results of Example 1, in which liquefied unpolished rice materials having initial Glc concentrations of 0.4 to 1.1% (w/v) were prepared for culturing *Lacticaseibacillus paracasei* YIT 9029 (*Lacticaseibacillus paracasei* YIT 9029 is sometimes referred to as LcS below) at 37°C and in which the changes in the viable cell counts, the Glc concentrations, the pH, and the lactic acid concentrations were examined.

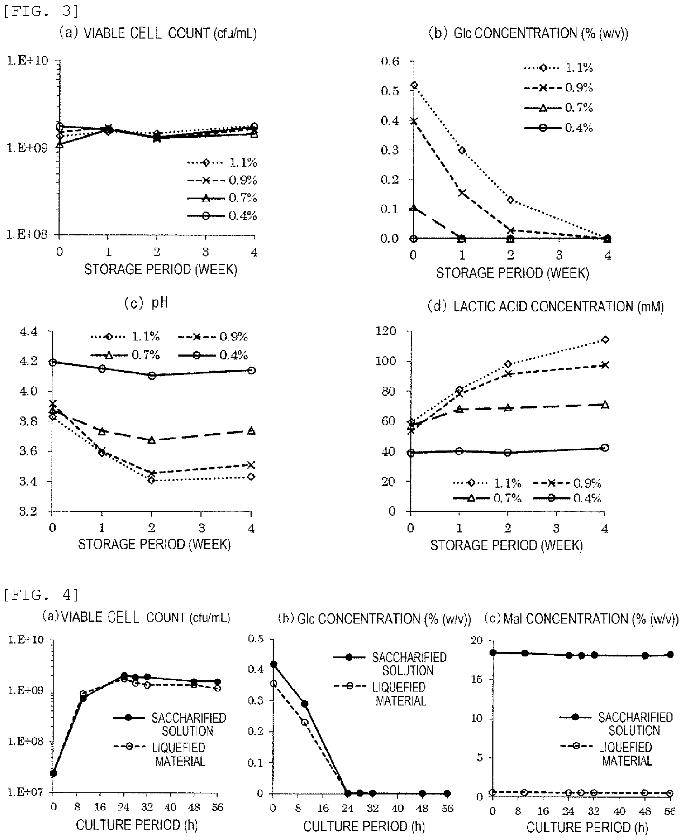

[Fig. 3] Fig. 3 shows the results of Example 2, in which LcS was cultured in liquefied unpolished rice materials having initial Glc concentrations of 0.4 to 1.1% (w/v) at 37°C for 24 hours and then stored at 10°C for four weeks and in which the changes in the viable cell counts, the Glc concentrations, the pH, and the lactic acid concentrations were examined.
[Fig. 4] Fig. 4 shows the results of Example 3, in which LcS was cultured in a saccharified material of unpolished rice (about 0.4% (w/v) Glc and about 18% (w/v) Mal) and a liquefied material before saccharification (about 0.4% (w/v) Glc) at 37°C and in which the changes in the viable cell counts, the Glc concentrations, and the Mal concentrations were compared.

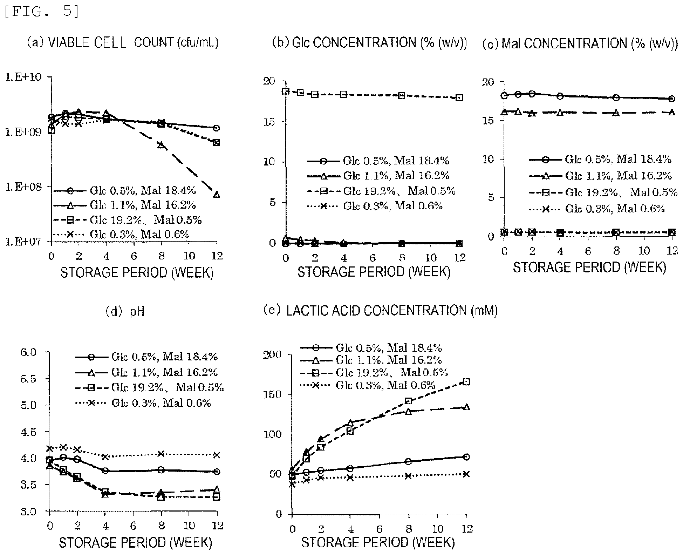

[Fig. 5] Fig. 5 shows the results of Example 4, in which LcS was cultured in various saccharified unpolished rice materials having different sugar concentrations at 37°C for 24 hours and then stored at 10°C for 12 weeks and in which the changes in the viable cell counts, the sugar concentrations, the pH, and the lactic acid concentrations were compared.

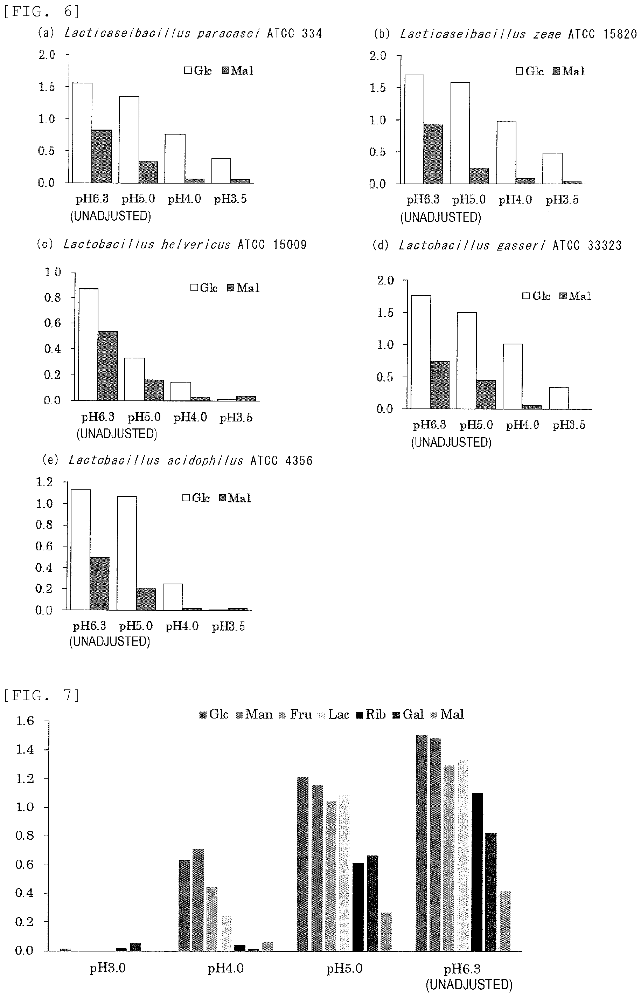

[Fig. 6] Fig. 6 shows the sugar consumptions of lactic acid bacteria which were cultured in a Glc-ILS medium for 24 hours and then re-cultured for 24 hours in Glc-ILS media and Mal-ILS media which were adjusted to various pH values in Example 5.
[Fig. 7] Fig. 7 shows the results of the pH sensitivities of assimilation of the sugars by LcS in Example 6.

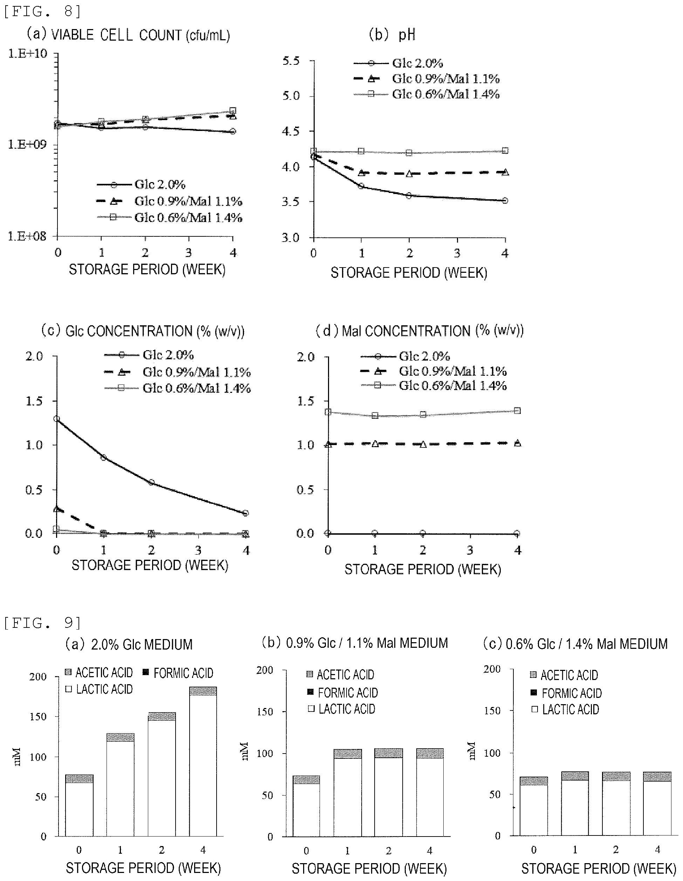

[Fig. 8] Fig. 8 shows the low-temperature storage stability of the LcS culture solutions in Example 7.
[Fig. 9] Fig. 9 shows the organic acid production of the LcS culture solutions during low-temperature storage in Example 7.

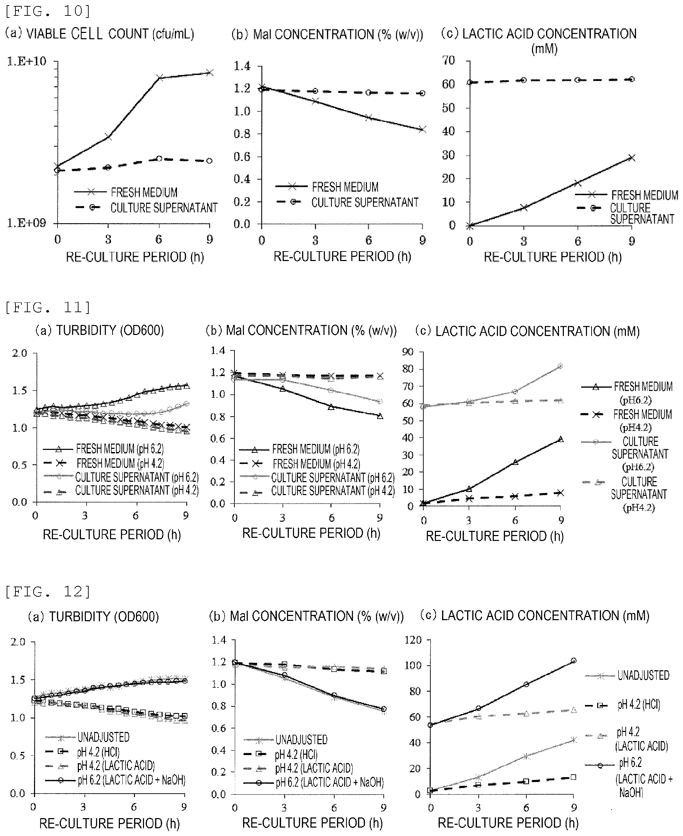

[Fig. 10] Fig. 10 shows the results of Example 8, in which LcS was cultured in an ILS medium with 0.6% (w/v) Glc/1.4% (w/v) Mal and in which the bacterial cells which were recovered/washed after assimilation/depletion of Glc were re-cultured in a Mal-ILS medium (a fresh medium, pH 6.2) or the culture supernatant (pH 4.2) after the recovery of the bacterial cells each in the same volume as that of the original culture solutions.
[Fig. 11] Fig. 11 shows the results of Example 8, in which LcS was cultured in an ILS medium with 0.6% (w/v) Glc/1.4% (w/v) Mal and in which the bacterial cells which were recovered/washed after assimilation/depletion of Glc were re-cultured in a Mal-ILS medium (a fresh medium, pH 6.2), a fresh medium which was adjusted to pH 4.2 with hydrochloric acid, the culture supernatant (pH 4.2), or the culture supernatant which was adjusted to pH 6.2 with sodium hydroxide each in the same volume as that of the original culture solutions.
[Fig. 12] Fig. 12 shows the results of Example 8, in which LcS was cultured in an ILS medium with 0.6% (w/v) Glc/1.4% (w/v) Mal and in which the bacterial cells which were recovered/washed after assimilation/depletion of Glc were re-cultured using a Mal-ILS medium (pH 6.2) having an unadjusted pH, a Mal-ILS medium which was adjusted to pH 4.2 with hydrochloric acid, a Mal-ILS medium which was adjusted to pH 4.2 with lactic acid, or a Mal-ILS medium which was adjusted to pH 4.2 with lactic acid and then returned to pH 6.2 with sodium hydroxide each in the same volume as that of the original culture solutions.

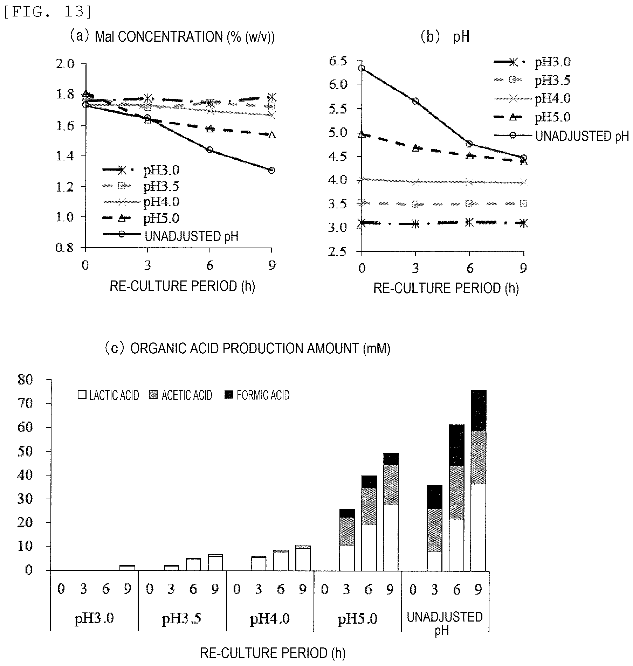

[Fig. 13] Fig. 13 shows the results of Example 9, in which LcS was cultured in a Glc-ILS medium for 14 hours and in which the bacterial cells which were recovered/washed were re-cultured in Mal-ILS media having medium pH of 3.0, 3.5, 4.0, and 5.0 and an unadjusted pH (pH 6.3) each in the same volume as that of the original culture solutions.

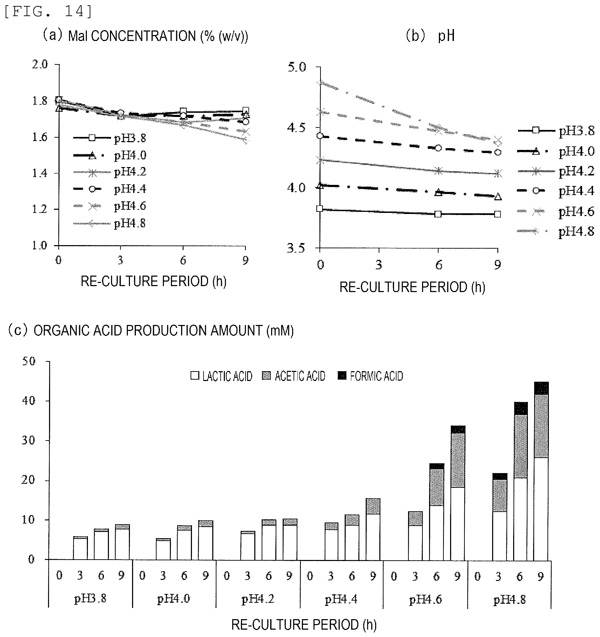

[Fig. 14] Fig. 14 shows the results of Example 9, in which LcS was cultured in a Glc-ILS medium for 14 hours and in which the bacterial cells which were recovered/washed were re-cultured in Mal-ILS media having medium pH of 3.8, 4.0, 4.2, 4.4, 4.6, and 4.8 each in the same volume as that of the original culture solutions.

### Description of Embodiments

The fermented food of the invention is a fermented food containing a lactic acid bacterium, the fermented food is substantially free from glucose, the fermented food contains sugars which can be assimilated by the lactic acid bacterium other than glucose, the sugars which can be assimilated by the lactic acid bacterium is derived from a vegetable material containing starch, and the fermented food has a pH of 4.5 or lower.

The lactic acid bacterium contained in the fermented food of the invention is not particularly limited, but examples thereof include lactic acid bacteria of the genus *Lacticaseibacillus* such as *Lacticaseibacillus paracasei* and *Lacticaseibacillus zeae* and those of the genus *Lactobacillus* such as *Lactobacillus helveticus, Lactobacillus acidophilus,* and *Lactobacillus gasseri.* One kind of the lactic acid bacteria or a combination of two or more kinds thereof can be used, but the genus *Lacticaseibacillus* and/or the genus *Lactobacillus* are preferable, and *Lacticaseibacillus paracasei* is more preferable. *Lacticaseibacillus paracasei* strain YIT 9029 (FERM BP-1366) is particularly preferable.

*Lacticaseibacillus paracasei* strain YIT 9029 listed above was deposited for an international deposit as *Lactobacillus casei* YIT 9029 (FERM BP-1366, accession date of May 1, 1981) to an international depositary authority under the Budapest Treaty, International Patent Organism Depositary, National Institute of Technology and Evaluation (#120, 2-5-8 Kazusakamatari, Kisarazu-shi, Chiba 292-0818). In this regard, LcS was previously classified as *Lactobacillus casei* but reclassified as *Lacticaseibacillus paracasei* after the recent reclassification of the genus *Lactobacillus* (Zheng et al., A taxonomic note on the genus Lactobacillus: Description of 23 novel genera, emended description of the genus Lactobacillus Beijerinck 1901, and union of Lactobacillaceae and Leuconostocaceae. Int. J. Syst. Evol. Microbiol. 2020 Apr; 70(4) :2782-2858 DOI 10.1099/ijsem.0.004107). In the present specification, the classification of the lactic acid bacteria is according to the new classification classified based on the above document.

The lactic acid bacterium content of the fermented food of the invention is not particularly limited but is, for example, 1.0×10⁵ to 1.0×10¹⁰ cfu/mL, preferably 1.0×10⁶ to 1.0×10¹⁰ cfu/mL.

The fermented food of the invention is substantially free from glucose. The substantially free here is, for example, 0.1 mass/volume % (mass/volume % is shown by % (w/v) below) or lower, preferably 0.05% (w/v) or lower. The glucose is substantially not contained in the fermented food because a lactic acid bacterium is cultured in a medium containing glucose and sugars which can be assimilated by the lactic acid bacterium other than glucose until the medium becomes substantially free from glucose as described below.

The sugars which can be assimilated by the lactic acid bacterium contained in the fermented food of the invention is preferably sugars derived from a vegetable material containing starch. In this regard, the sugars derived from a vegetable material containing starch here refers to sugars composing a vegetable material containing starch or sugars obtained by degradation or the like of a vegetable material containing starch with an enzyme or the like. The vegetable material containing starch is not particularly limited, but examples thereof include rice (unpolished rice), wheat, corn, potato, and the like. One kind of the vegetable materials containing starch or a combination of two or more kinds thereof can be used, but rice is preferable. Moreover, sugars derived from the vegetable materials are maltose, isomaltose, maltotriose, panose, and the like. One kind of the sugars derived from the vegetable materials or a combination of two or more kinds thereof can be used, but maltose is preferable.

The amount of the sugars which can be assimilated by the lactic acid bacterium derived from the vegetable material containing starch contained in the fermented food of the invention is not particularly limited but is, for example, 1 to 25% (w/v), preferably 10 to 25% (w/v).

The fermented food of the invention has a pH of 4.5 or lower, preferably a pH of 3.0 or higher and 4.5 or lower, more preferably 3.5 or higher and 4.4 or lower, particularly preferably 3.5 or higher and lower than 4.0. Because the fermented food is substantially free from glucose, contains sugars which can be assimilated by the lactic acid bacterium other than glucose, in which the sugars which can be assimilated by the lactic acid bacterium is derived from a vegetable material containing starch, and has a pH of 4.5 or lower, the lactic acid bacterium does not assimilate the sugars which the lactic acid bacterium otherwise assimilates. As a result, the fermented food can be stored stably for a long time because the acid production by the lactic acid bacterium can be inhibited and the flavor and the viability of the lactic acid bacterium can be improved. Here, that the fermented food can be stored stably for a long time means that the changes in the viable cell count and the acidity after storage at 10°C for three months are within ±50% or less of those at the start of the storage.

The fermented food of the invention may contain, for example, an aroma, a thickener, a coagulant, a colorant, fruit juice, or the like as long as the effects of the invention are not impaired.

The form of the fermented food of the invention is not particularly limited, and examples thereof include a drink, a solid yogurt-like food, a sauce, and the like.

In particular, when only sugars derived from a vegetable material containing starch is used as the sugars which can be assimilated by the lactic acid bacterium, the fermented food of the invention can be prepared as a fermented food in which milk and the specific allergen ingredients are not used and which does not contain sugars other than the sugars derived from a vegetable material containing starch, and thus the fermented food is "free from milk, the 28 specific allergen ingredients and added sugar". Furthermore, when maltose derived from a vegetable material containing starch is used as the sugars which can be assimilated by the lactic acid bacterium, a sufficient flavor is obtained without addition of a sweetener.

In this regard, the same effects are obtained when the fermented food of the invention is a fermented food containing a lactic acid bacterium which is substantially free from glucose, contains sugars which can be assimilated by the lactic acid bacterium other than glucose, in which the sugars which can be assimilated by the lactic acid bacterium is maltose, and has a pH of 4.5 or lower instead of the above configuration.

In this case, it is preferable that maltose is not derived from a vegetable material and that maltose which is commercially available or the like is added alone. The maltose content of the fermented food may be the same as that of the sugars which can be assimilated by the lactic acid bacterium other than glucose in the fermented food of the invention. Moreover, the fermented food may be the same as the fermented food of the invention except that the sugars which can be assimilated by the lactic acid bacterium other than glucose is maltose which is not derived from a vegetable material.

The fermented food of the invention explained above can be produced by a production method including the following steps (a) and (b).

A step (a) of culturing a lactic acid bacterium in a medium containing glucose and sugars which can be assimilated by the lactic acid bacterium other than glucose until the medium becomes substantially free from glucose.

A step (b) of adjusting the pH to 4.5 or lower when the pH after the termination of the culture is higher than 4.5.

The sugars which can be assimilated by the lactic acid bacterium other than glucose used in the step (a) refers to sugars composing a vegetable material containing starch or sugars obtained by degradation or the like of a vegetable material containing starch with an enzyme or the like. The vegetable material containing starch is not particularly limited, but examples thereof include rice (unpolished rice), wheat, corn, potato, and the like. One kind of the vegetable materials containing starch or a combination of two or more kinds thereof can be used, but rice is preferable. Moreover, sugars derived from the vegetable materials are maltose, isomaltose, maltotriose, panose, and the like. One kind of the sugars derived from the vegetable materials or a combination of two or more kinds thereof can be used, but maltose is preferable.

The glucose content of the medium is not particularly limited as long as it is such an amount that glucose is assimilated by the lactic acid bacterium and is substantially not contained in the final product, but the glucose content is, for example, 0.3 to 1.0% (w/v), preferably 0.3 to 0.6% (w/v).

The amount of the sugars which can be assimilated by the lactic acid bacterium other than glucose contained in the medium is not particularly limited but is, for example, 1 to 25% (w/v), preferably 10 to 25% (w/v).

The medium containing glucose and sugars which can be assimilated by the lactic acid bacterium other than glucose used in the step (a) is not particularly limited, but for example, one obtained by adding the glucose above and the sugars which can be assimilated by the lactic acid bacterium other than glucose to, for example, a medium such as soy milk and vegetable juice can be used, or one in combination with a component such as yeast extract can also be used. Moreover, a saccharified material obtained by a liquefaction treatment and a saccharification treatment of a vegetable material containing starch, a mixture of two or more saccharified materials having different sugar compositions obtained by the liquefaction treatment and the saccharification treatment of a vegetable material containing starch under two or more types of condition, a liquefied material obtained by the liquefaction treatment of a vegetable material containing starch, and the like can also be used. Of the media, the saccharified material obtained by the liquefaction treatment and the saccharification treatment of a vegetable material containing starch or the mixture of two or more saccharified materials having different sugar compositions obtained by the liquefaction treatment and the saccharification treatment of a vegetable material containing starch under two or more types of condition is preferable.

Of the media described above, the saccharified material obtained by the liquefaction treatment and the saccharification treatment of a vegetable material containing starch can be obtained as follows. First, a liquefaction enzyme, *koji,* molt, or the like for degrading and liquefying starch is caused to act on a vegetable material containing starch which has been subjected to crushing or the like when necessary, and thus a liquefied material is obtained. Examples of the liquefaction enzyme include various types of α-amylase. One kind of liquefaction enzyme or a combination of two or more kinds thereof can be used, but heat-resistant α-amylase is preferable.

The conditions of the liquefaction treatment of the vegetable material containing starch may be appropriately set depending on the liquefaction enzyme used or the like and are, for example, in the case of α-amylase, conditions of the addition amount of 0.001 to 0.5% (w/v), preferably 0.01 to 0.1% (w/v) based on the vegetable material containing starch, at 65 to 95°C, preferably at 75 to 90°C, for 10 minutes to four hours, preferably for 30 minutes to an hour. As a result, a liquefied material is obtained. After the termination of the liquefaction treatment, the liquefaction enzyme or the like may be inactivated by heating.

After the termination of the liquefaction treatment, the saccharification treatment is conducted. A saccharification enzyme which degrades the liquefied material into sugars is used for the saccharification treatment. Examples of the saccharification enzyme include β-amylase, pullulanase, glucoamylase, and the like. One kind of the saccharification enzymes or a combination of two or more kinds thereof can be used, but the combination of β-amylase and pullulanase is preferable.

The conditions of the saccharification treatment of the liquefied material may be appropriately set depending on the saccharification enzyme used and are, for example, in the case of β-amylase and pullulanase, conditions of the addition amounts of 0.01 to 0.1% (w/v) based on the liquefied material at 50 to 70°C for four to 36 hours. As a result, a saccharified material is obtained. After the termination of the saccharification treatment, the saccharification enzyme may be inactivated by heating.

The glucose amount in the saccharified material may be the same as the content in the medium described above, and one skilled in the art can achieve such an amount by adjusting the conditions of the liquefaction treatment and the saccharification treatment.

The saccharified material obtained by the liquefaction treatment and the saccharification treatment of a vegetable material containing starch can be obtained, for example, by treating unpolished rice as follows. After suspending unpolished rice flour and water, the suspension is heated to a range in which the suspension does not gelatinize, for example, to around 50°C to 60°C, with stirring. The mixing ratio of the unpolished rice flour and water may be appropriately set, but the solid content is preferably around 10 to 35 mass%. Next, heat-resistant α-amylase is added, and the mixture is heated and liquefied. The glucose content of the saccharified material to be obtained in the end is adjusted by appropriately setting the addition amount of the liquefaction enzyme and the reaction temperature and the period of the liquefaction within the condition range described above. After the termination of the reaction, the mixture is immediately heated for inactivation of the liquefaction enzyme and sterilization. Next, β-amylase and pullulanase are added as the saccharification enzymes, and the reaction is conducted under the conditions. After the termination of the reaction, the mixture is immediately heated for both inactivation of the saccharification enzymes and sterilization. As a result, a saccharified material of unpolished rice having the same glucose content as that of the medium above and a maltose content of around 20% (w/v) can be prepared. By appropriately adjusting the conditions of the liquefaction treatment and the saccharification treatment, a saccharified material can be obtained also from a vegetable material other than unpolished rice, such as grains including polished rice and wheat and potatoes.

Of the media described above, the mixture of two or more saccharified materials having different sugar compositions obtained by the liquefaction treatment and the saccharification treatment of a vegetable material containing starch under two or more types of condition can be obtained as follows. First, saccharified materials obtained by the liquefaction treatment and the saccharification treatment of the vegetable material containing starch described above are obtained separately in the condition range described above, and two or more saccharified materials having different sugar compositions are obtained. Then, the two or more saccharified materials having different sugar compositions are mixed, and thus a final saccharified material is obtained. The glucose amount in the final saccharified material may be the same as the content in the medium described above, and one skilled in the art can achieve such an amount by adjusting the conditions of the liquefaction treatment and the saccharification treatment.

When unpolished rice flour is used as the vegetable material containing starch, the mixture of two or more saccharified materials having different sugar compositions obtained by the liquefaction treatment and the saccharification treatment of the vegetable material containing starch under two or more types of condition can be obtained by treating unpolished rice as follows. After heat-resistant α-amylase and unpolished rice flour are added to water heated to 75°C to 90°C in advance, the mixture is liquefied with stirring. A slurry obtained by dissolving unpolished rice flour in a small amount of water may be added instead of the unpolished rice flour. The addition amount of the unpolished rice flour may be appropriately set but is preferably set in such a manner that the solid content of the final saccharified material becomes around 10 to 35% (w/v). Next, by adding β-amylase and pullulanase as the saccharification enzymes to the liquefied material and conducting the reaction under the conditions, a saccharified material having a glucose content of around 0.1% (w/v) and a maltose content of around 20% (w/v) can be prepared. Moreover, by adding glucoamylase as the saccharification enzyme to the liquefied material and conducting the reaction under the conditions, a saccharified material having a glucose content of around 22% (w/v) and a maltose content of 0.5% (w/v) can be prepared. A saccharified material of unpolished rice having the same glucose content as that of the medium above and a maltose content of around 20% (w/v) can be prepared by mixing the saccharified materials. By appropriately adjusting the conditions of the liquefaction treatment and the saccharification treatment, a saccharified material can be obtained also from a vegetable material other than unpolished rice, such as grains including polished rice and wheat and potatoes.

Of the media described above, the liquefied material obtained by the liquefaction treatment of a vegetable material containing starch or the saccharified material obtained by the saccharification treatment of a vegetable material containing starch can be obtained by appropriately employing the liquefaction treatment or the saccharification treatment described above using the vegetable material containing starch.

The lactic acid bacterium is cultured in the medium described above until the medium becomes substantially free from glucose. The conditions for fermenting until the lactic acid bacterium assimilates substantially all the glucose contained in the medium are not particularly limited, but for example, the lactic acid bacterium is inoculated at a percentage of 0.001 to 5.0%, preferably 0.01 to 1.0% based on the medium and cultured at 25 to 37°C, preferably at 30 to 37°C, for 24 to 120 hours, preferably for 24 to 48 hours.

After culturing the lactic acid bacterium in the medium until the medium becomes substantially free from glucose as described above, the pH is adjusted to 4.5 or lower when the pH after the termination of the culture is higher than 4.5. Here, the pH after the termination of the culture can also be adjusted by adjusting the glucose concentration of the saccharified material. By adjusting the pH to 4.4 or lower, preferably lower than 4.0, assimilation of the sugars which can be assimilated by the lactic acid bacterium other than glucose can be inhibited during storage, and stable storage is possible. Here, for example, lactic acid, acetic acid, citric acid, or the like may be added separately to adjust the pH.

After the termination of the fermentation, treatment such as homogenization or an operation such as dilution, flavor adjustment, and filling may be appropriately conducted in the range in which the pH and the glucose concentration are not affected.

As explained above, according to the invention, because the fermented food containing a lactic acid bacterium is substantially free from glucose, contains sugars which can be assimilated by the lactic acid bacterium other than glucose, and has a pH of 4.5 or lower, assimilation of the sugars which can be assimilated by the lactic acid bacterium other than glucose during storage of the fermented food can be inhibited.

Moreover, according to the invention, because the fermented food containing a lactic acid bacterium is substantially free from glucose, contains sugars which can be assimilated by the lactic acid bacterium other than glucose, and has a pH of 4.5 or lower, a decrease in the pH during storage of the fermented food can be inhibited.

### Examples

The invention is explained in detail below referring to Examples, but the invention is not limited at all to the Examples.

### Reference Example 1

### Preparation of Saccharified Materials:

RO water in an amount of 400 g was poured into a 1-L flask and heated to 85°C with a hot stirrer having a solution temperature sensor. After dissolving 45 g of unpolished rice flour therein, a liquefaction enzyme was added, and 125 g of unpolished rice flour and 30 g of RO water were further added. The mixture was stirred at the same temperature for 30 minutes. The liquefaction enzyme (Kleistase E5CC, Amano Enzyme Inc.) was added to a final concentration of 0.05% (w/v). After the termination of the liquefaction, the mixture was immediately heated for 30 minutes in a boiling water bath at 100°C, and thus inactivation of the enzyme and sterilization were conducted. Various saccharification enzymes were caused to act on the liquefied material, and thus saccharified materials containing a large amount of maltose (Mal) or glucose (Glc) were prepared. To produce Mal, β-amylase (β-amylase F "Amano", Amano Enzyme Inc.) and pullulanase (Kleistase PL45, Amano Enzyme Inc.) were used, and glucoamylase (Gluczyme NL4.2, Amano Enzyme Inc.) was used to produce Glc. After the termination of the saccharification, the mixtures were immediately heated for 30 minutes in a hot water bath at 100°C, and thus inactivation of the enzymes and sterilization were conducted. The two kinds of saccharified materials were mixed, and it was attempted to prepare saccharified materials having predetermined Glc concentrations.

From the saccharification treatments, a saccharified material having a low Glc concentration and a high Mal concentration (21% (w/v) Mal and lower than 0.1% (w/v) Glc) and a saccharified material having a high Glc concentration (22% (w/v) Glc and 0.5% (w/v) Mal) could be prepared. By mixing the two, saccharified unpolished rice materials having various Glc concentrations in the range of 0.1% to 1.0% could be prepared (Fig. 1a). The Mal concentrations of all the saccharified materials were around 20% (w/v) (Fig. 1b).

### Example 1

### Initial Glc Concentrations and Fermentation Properties:

Unpolished rice flour was liquefied under four types of condition with different amounts of the enzyme, different reaction temperatures, and different reaction periods for the liquefaction, and liquefied materials having Glc concentrations of 0.4, 0.7, 0.9, and 1.1% (w/v) were prepared. As a preculture solution of *Lacticaseibacillus paracasei* strain YIT 9029 (FERM BP-1366) (hereinafter referred to "LcS"), a bacterial solution obtained by inoculating an inoculum of LcS at 1.0% in the ILS medium shown in Table 1 below (sometimes referred to Glc-ILS medium) and culturing at 37°C for 24 hours was used. The preculture solution was inoculated at 0.1% in the liquefied materials and cultured at 37°C for 24 hours. Next, the fermented materials were transferred at 1.0% to fresh liquefied unpolished rice materials and cultured at 37°C, and the viable cell counts, the pH, the Glc concentrations, and the lactic acid concentrations were measured.

**[Table 1]**

| Component | Amount (per 1000 g) |
|---|---|
| Trypticase peptone | 10.00g |
| Yeast extract | 5.00g |
| Tryptose | 3.00g |
| K₂PO₄ | 2.98g |
| KH₂PO₄ | 3.00g |
| Diammonium hydrogen citrate | 2.00g |
| Tween 80 | 1.00g |
| CH₃COONa·3H₂O | 1.70g |
| L-Cystein HCl·H₂O | 0.20g |
| Glucose | 20.00g |
| Salt solution* | 5.00mL |

| | |
|---|---|
| *A solution obtained by dissolving 11.5 g of MgSO₄·7H₂O, 3.09 g of MnSO₄·5H₂O, and 0.68 g of FeSO₄·7H₂O in 100 mL of RO water. | |

The viable cell counts of all the liquefied materials reached 1.6×10⁹ cfu/mL or higher (Fig. 2a). In the liquefied unpolished rice material with an initial Glc of 0.4% (w/v), depletion of Glc was observed at the 24th hour of the culture, and the decrease in the pH and the production of lactic acid stopped at the same time point (Figs. 2b to d). On the other hand, in the liquefied unpolished rice materials with initial Glc of 0.7% (w/v) or higher, even after reaching the stationary phase, Glc remained, and the production of lactic acid continued (Figs. 2b to d. It was thus shown that Glc can be assimilated/depleted by LcS as a growth substrate by adjusting the Glc concentration of the liquefied material. Moreover, it was found that the decrease in the pH and the production of lactic acid after reaching the stationary phase can be inhibited by depletion of Glc.

### Example 2

### Stability of LcS Fermented Materials of Liquefied Materials in Low-Temperature Storage:

LcS was cultured at 37°C for 24 hours in various liquefied unpolished rice materials by the same method as that of Example 1. Next, the culture solutions were inoculated at 1.0% to fresh liquefied materials and cultured at 37°C for 24 hours. After the culture, the culture solutions were stored at 10°C for four weeks, and the viable cell counts, the pH, the Glc concentrations, and the lactic acid concentrations were measured over time.

At the start of the storage, depletion of Glc was observed only in the liquefied unpolished rice material having an initial Glc concentration of 0.4% (w/v) (Fig. 3b). The pH and the lactic acid concentration of the liquefied material were almost constant until the fourth week of the storage (Figs. 3c to d). On the other hand, the production of lactic acid continued in the fermented materials in which Glc remained at the start of the storage (Fig. 3d). The viable cell counts were almost constant from the start of the storage to the fourth week of the storage regardless of the initial Glc concentration, and all the fermented materials showed high viability (Fig. 3a). From the above results, it was shown that the acid production can be inhibited not only in the stationary phase but also during low-temperature storage by depleting Glc in the liquefied material.

### Example 3

### Fermentation Properties of Saccharified Unpolished Rice Material:

A liquefied unpolished rice material was prepared in the same manner as in Example 1 and saccharified using β-amylase (β-amylase F "Amano", Amano Enzyme Inc.) and pullulanase (Kleistase PL45, Amano Enzyme Inc.), and thus a saccharified unpolished rice material (about 0.4% (w/v) Glc and about 18% (w/v) Mal) was prepared. The growth of LcS in the saccharified material and that in the liquefied material before the saccharification (about 0.4% (w/v) Glc) were compared.

The highest bacterial count of LcS reached in the saccharified unpolished rice material was 2.0×10⁹ cfu/mL, which was equivalent to that of the culture in the liquefied unpolished rice material before the saccharification (Fig. 4a). Moreover, although depletion of Glc was observed at the 24th hour of the culture as in the liquefied unpolished rice material, the Mal concentration did not change even after the Glc depletion (Figs. 4b and c). Thus, it was found that LcS assimilates Glc preferentially in a saccharified unpolished rice material containing Glc and Mal. Moreover, because the concentration of Mal produced by the saccharification did not change, the possibility of functioning as a sweetener was shown.

### Example 4

### Stability of Fermented Saccharified Materials in Low-Temperature Storage:

Various saccharified unpolished rice materials having different Glc and Mal concentrations were prepared in the same manners as in Example 1 and Example 3, and LcS was cultured at 37°C for 24 hours. After the termination of the culture, the fermented materials were stored at 10°C, and the sugar concentrations, the lactic acid concentrations, and the viable cell counts were examined after one week, two weeks, four weeks, eight weeks, and 12 weeks.

The decrease in the pH and the production of lactic acid continued also during the storage period in the fermented materials of two kinds of saccharified material (1.1% (w/v) Glc and 16.2% (w/v) Mal, and 19.2% (w/v) Glc and 0.5% (w/v) Mal) in which Glc remained at the start of the storage, and the viable cell counts decreased after the fourth week or the eighth week of the storage (Figs. 5a, b, d, and e). On the other hand, it was observed that Glc was depleted at the start of the storage in the saccharified materials with 0.3% (w/v) Glc and 0.6% (w/v) Mal, and 0.5% (w/v) Glc and 18.4% (w/v) Mal (Fig. 5b). It was shown that the decrease in the pH and the production of lactic acid during the storage period were inhibited in the fermented materials of the saccharified materials (Figs. 5d and e). Moreover, the decreases in the viable cell counts during the storage period were mild compared to those of the other two kinds of fermented material. The viable cell counts also at the 12th week of the storage were 1.2×10⁹ cfu/mL, and the viabilities were both around 60% (Fig. 5a). Almost no change was observed in the Mal concentrations of all the fermented materials during the storage period (Fig. 5c). Accordingly, it was found that the acid production during low-temperature storage can be inhibited by regulating the Glc concentration in the saccharified unpolished rice material. Moreover, it was also found that the Mal concentration can be maintained almost without any change and that the viable cell count can be maintained high over a long period of time. Therefore, the results suggest the possibility that a LcS fermented drink which is free of added sugar and which has excellent storage stability can be provided using the fermentation substrate.

### Example 5

### pH and Mal Assimilation Properties by Other Lactic Acid Bacteria:

Five strains of five species of lactic acid bacteria (*Lactobacillus acidophilus* (ATCC4356), *Lactobacillus helveticus* (ATCC15009), *Lacticaseibacillus paracasei* (ATCC334), *Lactobacillus gasseri* (ATCC33323), and *Lacticaseibacillus zeae* (ATCC15820)) were cultured for 24 hours in the Glc-ILS medium shown in Table 1 of Example 1 and then re-cultured for 24 hours in Glc-ILS media or Mal-ILS media (obtained by changing Glc from 2% (w/v) to 0.6% (w/v) and newly adjusting Mal to 1.4% (w/v) in the Glc-ILS medium shown in Table 1) which were adjusted to various pH values, and the influences of the pH of the medium on the assimilation of Mal and Glc were compared. Here, strain ATCC can be purchased from ATCC (American Type Culture Collection 10801 University Boulevard Manassas, Virginia 20110-2209 U.S.A).

It was found that the pH sensitivities of Mal assimilation of the five strains were also higher than that for Glc like LcS (Fig. 6). From the above results, it is believed that Mal assimilation can be inhibited by adjusting the pH also for lactic acid bacteria other than LcS.

### Example 6

### Influences of pH on Sugar Source Assimilation:

LcS was cultured in an ILS medium containing 2.0% (w/v) Glc, and washed bacterial cells were prepared. The bacterial cells were re-cultured in fresh ILS media containing various sugars each in the same volume as that of the original medium, and the assimilation properties were examined. The sugar sources of the ILS media used for the re-culture were Glc, Mal, galactose (Gal), fructose (Fru), mannose (Man), ribose (Rib), or lactose (Lac), and the pH was adjusted to pH 3.0, 4.0, or 5.0 with lactic acid.

The assimilation of all the sugars was inhibited in a pH-dependent manner (Fig. 7). However, although the assimilation of Mal, Gal, and Rib stopped under the conditions of pH 4.0 or lower, the assimilation of Glc, Fru, Man, and Lac stopped only at pH 3.0 (Fig. 7). Accordingly, it was found that the threshold of the pH of the medium at which the sugar can be assimilated differs with the kind of the sugar and that the pH sensitivities of the assimilation of Mal, Gal, and Rib are higher than those of the other sugars.

### Example 7

### Storage Stability of Fermented Materials with Glc Depletion:

To evaluate the stability of fermented materials in which Glc was depleted in low-temperature storage, LcS was cultured for 14 hours in media with 2.0% (w/v) Glc, 0.9% (w/v) Glc/1.1% (w/v) Mal, and 0.6% (w/v) Glc/1.4% (w/v) Mal and then stored at 4°C for four weeks, and the viable cell counts, the pH, the organic acid concentrations, and the residual sugars were measured.

It was found that, while Glc remained in the media with 2.0% (w/v) Glc and 0.9% (w/v) Glc/1.1% Mal at the start of the storage, Glc was almost depleted in the medium with 0.6% (w/v) Glc/1.4% (w/v) Mal (Fig. 8c). In the medium with 2.0% Glc, Glc was assimilated also during the storage period, and the decrease in the pH and the production of lactic acid continued (Figs. 8b and c, and Fig. 9a). On the other hand, it was found that, in the other two kinds of medium, the decrease in the pH stopped with the depletion of Glc and that the pH was maintained stably until the termination of the storage (Figs. 8b and c). The organic acid concentrations did not change after the depletion of Glc, and results which were consistent with the change in the pH were obtained (Figs. 9b and c). Moreover, the Mal concentrations in the media did not change throughout the storage period (Fig. 8d). During the low-temperature storage period, although no large change was observed in the viable cell counts, the viable cell counts at the fourth week of the storage in the media with 0.9% (w/v) Glc/1.1% (w/v) Mal and 0.6% (w/v) Glc/1.4% (w/v) Mal were higher than that of the medium with 2.0% (w/v) Glc (Fig. 8a). From the above results, it was found that the acid production can be inhibited also in low-temperature storage by depleting Glc. This suggests the possibility that a drink with a small change in the flavor during the storage period can be produced by depletion of Glc.

### Example 8

### Mal Assimilation-Inhibiting Factor in ILS Media:

The factor responsible for inhibition of Mal assimilation after Glc depletion was examined. First, LcS was cultured at 37°C for 14 hours using an ILS medium containing 0.6% (w/v) Glc and 1.4% (w/v) Mal, and the bacterial cells in which Mal assimilation after Glc depletion was inhibited were recovered by centrifugation. The recovered bacterial cells were washed with saline and then re-cultured in various ILS media or the culture supernatant recovered during the recovery of the bacterial cells each in the same volume as that of the original culture solution, and the sugar assimilation properties, the changes in the pH, and the organic acid production were examined.

Through re-culture in a fresh ILS medium containing 1.4% (w/v) Mal (Mal-ILS medium, pH 6.2), the inhibition of Mal assimilation was removed, and bacterial cell growth and acid production were observed (Fig. 10). On the other hand, in the case of re-culture in the culture supernatant (pH 4.2) recovered during the recovery of the bacterial cells, Mal assimilation and production of lactic acid were not observed (Figs. 10b and c). The inhibition of Mal assimilation and lactic acid production was removed when the culture supernatant was neutralized (pH 6.2) with sodium hydroxide (Figs. 11b and c). Moreover, through re-culture in fresh Mal-ILS media which were adjusted to pH 4.2 by adding lactic acid or hydrochloric acid, the Mal assimilation and the production of lactic acid were inhibited (Figs. 12b and c). The inhibition was removed through neutralization of the pH (pH 6.2) with sodium hydroxide (Figs. 2b to d). These results show that Mal assimilation is inhibited by a decrease in the pH of the medium.

### Example 9

### pH Range Required for Inhibition of Mal Assimilation in ILS Medium:

LcS was cultured at 37°C for 14 hours using an ILS medium containing 2.0% (w/v) Glc, and bacterial cells were recovered by centrifugation and washed with saline. The bacterial cells were suspended and re-cultured in 2.0% (w/v) Mal-containing ILS media which were adjusted to various pH values each in the same volume as that of the original culture solution, and the residual sugar concentrations, the pH, and the organic acid concentrations were measured over time. The initial pH values of the Mal-ILS media were adjusted to 3.0, 3.5, 3.8, 4.0, 4.2, 4.4, 4.6, 4.8, and 5.0 with lactic acid.

Re-culture was conducted using 2.0% (w/v) Mal-containing ILS media which were adjusted to pH 3.0, 3.5, 4.0, and 5.0 with lactic acid and the same medium with an unadjusted pH (pH 6.3). As a result, Mal was assimilated in the medium having pH 5.0 and the medium with an unadjusted pH during the re-culture, and the pH decreased with time. On the other hand, in the media having pH 4.0 or lower, Mal was hardly assimilated, and no decrease in the pH was observed (Figs. 13a and b). Moreover, the organic acid production was inhibited considerably (Fig. 13c). Furthermore, as a result of examination with Mal-ILS media which were adjusted to pH 3.8, 4.0, 4.2, 4.4, 4.6, and 4.8 with lactic acid, while a decrease in the Mal concentration and a decrease in the pH were observed under the conditions of pH 4.6 or higher, no clear change was observed under the conditions of pH 4.4 or lower (Figs. 14a and b). The production amounts of the organic acids were almost constant under the conditions of pH 4.4 or lower (Fig. 14c). From the above results, it was found that the Mal assimilation by LcS is inhibited in a medium pH-dependent manner and almost stops by adjusting the pH to lower than 4.6 under the experimental conditions.

### Industrial Applicability

The invention is a new fermented food containing sugars derived from a vegetable material containing starch and is useful for promoting health.

## Claims

1. A fermented food comprising:
a lactic acid bacterium, wherein
the fermented food is substantially free from glucose,
the fermented food contains sugars which can be assimilated by the lactic acid bacterium other than glucose,
the sugars which can be assimilated by the lactic acid bacterium is derived from a vegetable material containing starch, and
the fermented food has a pH of 4.5 or lower.

2. The fermented food according to claim 1, wherein the sugars which can be assimilated by the lactic acid bacterium derived from the vegetable material containing starch is maltose.

3. The fermented food according to claim 1 or 2, wherein the lactic acid bacterium is one kind or two or more kinds selected from the genus *Lacticaseibacillus* and/or the genus *Lactobacillus.*

4. The fermented food according to claim 1 or 2, wherein the lactic acid bacterium is *Lacticaseibacillus paracasei.*

5. The fermented food according to claim 1 or 2, wherein the lactic acid bacterium is *Lacticaseibacillus paracasei* strain YIT 9029 (FERM BP-1366).

6. A method for producing a fermented food, the method comprising the following steps (a) and (b):
a step (a) of culturing a lactic acid bacterium in a medium containing glucose and sugars which can be assimilated by the lactic acid bacterium other than glucose until the medium becomes substantially free from glucose; and
a step (b) of adjusting the pH to 4.5 or lower when the pH after the termination of the culture is higher than 4.5.

7. The method for producing a fermented food according to claim 6, wherein the sugars which can be assimilated by the lactic acid bacterium other than glucose is derived from a vegetable material containing starch.

8. The method for producing a fermented food according to claim 6 or 7, wherein the sugars which can be assimilated by the lactic acid bacterium other than glucose is maltose.

9. The method for producing a fermented food according to any one of claims 6 to 8, wherein the medium containing glucose and the sugars which can be assimilated by the lactic acid bacterium other than glucose is a saccharified material obtained by a liquefaction treatment and a saccharification treatment of a vegetable material containing starch.

10. The method for producing a fermented food according to any one of claims 6 to 8, wherein the medium containing glucose and the sugars which can be assimilated by the lactic acid bacterium other than glucose is a mixture of two or more saccharified materials having different sugar compositions obtained by the liquefaction treatment and the saccharification treatment of a vegetable material containing starch under two or more types of condition.

11. The method for producing a fermented food according to any one of claims 6 to 10, wherein the lactic acid bacterium is of the genus *Lacticaseibacillus* and/or the genus *Lactobacillus.*

12. The method for producing a fermented food according to any one of claims 6 to 10, wherein the lactic acid bacterium is *Lacticaseibacillus paracasei.*

13. The method for producing a fermented food according to any one of claims 6 to 10, wherein the lactic acid bacterium is *Lacticaseibacillus paracasei* strain YIT 9029 (FERM BP-1366) .

14. A method for inhibiting assimilation of sugars which can be assimilated by a lactic acid bacterium other than glucose during storage of a fermented food containing a lactic acid bacterium which is **characterized in that**
the fermented food is substantially free from glucose,
contains the sugars which can be assimilated by the lactic acid bacterium other than glucose, and
has a pH of 4.5 or lower.

15. A method for inhibiting a decrease in a pH during storage of a fermented food containing a lactic acid bacterium which is **characterized in that**
the fermented food is substantially free from glucose,
contains sugars which can be assimilated by the lactic acid bacterium other than glucose, and
has a pH of 4.5 or lower.
